(19)

**Europäisches Patentamt**
**European Patent Office**
**Office européen des brevets**

(11) **EP 2 217 145 B1**

(12) **EUROPÄISCHE PATENTSCHRIFT**

(45) Veröffentlichungstag und Bekanntmachung des Hinweises auf die Patenterteilung:
**21.09.2011 Patentblatt 2011/38**

(21) Anmeldenummer: **08849589.0**

(22) Anmeldetag: **04.11.2008**

(51) Int Cl.:
*A61B 5/113* (2006.01) *A61B 5/08* (2006.01)

(86) Internationale Anmeldenummer:
**PCT/EP2008/009286**

(87) Internationale Veröffentlichungsnummer:
**WO 2009/062613 (22.05.2009 Gazette 2009/21)**

(54) **KLEIDUNGSSTÜCK ZUR ERFASSUNG EINER ATEMBEWEGUNG**

CLOTHING ARTICLE FOR DETECTING BREATHING MOVEMENT

PIECE D'HABILLEMENT A DETECTION DU MOUVEMENT RESPIRATOIRE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **12.11.2007 DE 102007053843**

(43) Veröffentlichungstag der Anmeldung:
**18.08.2010 Patentblatt 2010/33**

(73) Patentinhaber: **Fraunhofer-Gesellschaft zur Förderung der angewandten Forschung e.V.
80686 München (DE)**

(72) Erfinder:
• **TOBOLA, Andreas**
**91334 Hemhofen (DE)**
• **WEIGAND, Christian**
**90556 Cadolzburg (DE)**

(74) Vertreter: **Zinkler, Franz
Schoppe, Zimmermann, Stöckeler & Zinkler
Patentanwälte
Postfach 246
82049 Pullach bei München (DE)**

(56) Entgegenhaltungen:
EP-A- 1 731 094    DE-A1- 4 229 073
FR-A- 2 821 262    US-A- 5 454 376
US-B1- 6 341 504

EP 2 217 145 B1

**Beschreibung**

Die vorliegende Erfindung betrifft Vorrichtungen zur Überwachung von Änderungen eines Bewegungszustandes von Teilen eines Lebewesens, und insbesondere Vorrichtungen zur Erfassung von Atembewegungen eines Lebewesens.

Als Atmungsanstrengung wird die Variation des Körperumfangs an mindestens einer Stelle am Rumpf eines Lebewesens bezeichnet. Typischerweise wird die Atmungsanstrengung an zwei Stellen des Rumpfes, an Abdomen und Thorax, gemessen und als Signal abgebildet und gespeichert. Die Atmungsanstrengung bildet die An- bzw. Entspannung der Lungenmuskulatur ab. Eine Änderung der Atmungsanstrengung - also eine An- bzw. Entspannung der Lungenmuskeln - kann auch stattfinden, wenn tatsächlich kein Atemfluss, also kein Austausch von Atemgasen, vorhanden ist. In der Regel ist jedoch die Atmungsanstrengung mit dem Atemfluss verbunden.

Beispielsweise erlaubt eine graphische Abbildung der Signale der Atmungsanstrengung Ärzten eine direkte Interpretation eines Vitalzustandes eines Lebewesens bezüglich der Lungenfunktion. Zudem ist eine Weiterverarbeitung dieses Signals zu Vitalparametern möglich. Dies können z.B. Atemfrequenz, Atemvolumen, Atemtiefe oder der generelle Atmungsverlauf sein. Die Erfassung der Atmungsanstrengung kann Anwendung in der Somnologie (Schlafforschung), der Sportmedizin oder der Heimüberwachung finden. Ein Beispiel für eine Anwendung in der Heimüberwachung stellt das Phänomen des plötzlichen Kindstodes, in der medizinischen Literatur mit SIDS (Sudden Infant Death Syndrome) bezeichnet, dar. Grund für diesen plötzlichen Kindstod ist ein zentraler Atemstillstand unbekannter Ursache. Dieses Beispiel aus dem Bereich der Medizin ist insofern besonders relevant, als SIDS alle Kinder in ihrem ersten Lebensjahr bedroht. Der plötzliche Kindstod ist für fast die Hälfte aller Todesursachen zwischen dem zweiten und dem zwölften Lebensmonat verantwortlich.

Bekannte Verfahren zur Erfassung der Atmungsanstrengung beruhen beispielsweise auf Sensoren bzw. Messwertaufnehmern, die respiratorische Bewegungen aufnehmen, Geräten zur Messung der Thorax-Impedanz, Sensoren für Herztätigkeit, wie z.B. EKG-Geräte, Induktions-Plethysmographen oder Pulsoximetern. Bei einigen dieser Verfahren wird die Atmungsanstrengung mittels Bändern bzw. Gurten gemessen, die um eine Körperpartie angelegt werden. Die Methoden, mit der die Atmungsanstrengung ermittelt werden, lassen sich in zwei Klassen einordnen. Belastungsfreie Methoden und solche, die eine gewisse Spannung der Gurte erfordern. Als Beispiel für ein belastungsfreies Verfahren ist die Induktions-Plethysmographie zu nennen, bei der die Gurte locker um die ausgewählten Körperpartien angebracht werden. Im Gegensatz dazu ist bei der Plethysmographie der Atmung mittels Dehnungsmessstreifen (Strain-Gauge-Plethysmographie) eine weitaus größere Spannung der Gurte erforderlich, die für einen Patienten unangenehm sein kann. Unabhängig von der Messmethode können solche Gurte unter der Kleidung getragen werden, müssen jedoch darunter mittels Kabeln an eine Signalaufbereitungselektronik angeschlossen werden. Eine negative Eigenschaft der Gurte ist beispielsweise deren Verrutschen beim Tragen. Nach dem Anlegen werden die Bänder an einer bestimmten Stelle des Körpers positioniert, können dann jedoch ihre Position im Verlauf der Messung ändern, beispielsweise aufgrund von Bewegung oder Unebenheiten der jeweiligen Körperpartie.

Die EP-A-1731094 offenbart ein Kleidungsstück zur Erfassung einer Atembewegung. Das Kleidungsstück besteht aus einem elastischen, nicht leitenden Material. Ein um den Thorax verlaufender Atmungsinformationssensor in Brusthöhe ist aus einem in das Kleidungsstück eingearbeiteten leitfähigen Material gefertigt. Zusätzlich ist auch ein Atmungsinformationssensor in Bauchhöhe aus einem in das Kleidungsstück eingearbeiteten leitfähigen Material vorhanden. Die Atmungsinformationssensoren sind aus einem leitfähigen Material mit Metallpartikeln hergestellt. Um eine Atmungsbewegung zu erfassen, wird jeweils ein elektrischer Strom durch die Atmungsinformationssensoren geschickt.

Die US-B1-6341504 beschreibt ein elastisches Band aus einem elastischen Material, in das wenigstens ein starrer, elektrischer Draht sinusförmig eingearbeitet ist. Das Band kann zur Überwachung vorbestimmter physiologischer Funktionen, wie beispielsweise zur Überwachung einer Atembewegung eingesetzt werden. Dazu kann ein derartiges Band beispielsweise mit einem Kleidungsstück kombiniert werden. Das gezeigte Kleidungsstück umfasst ein erstes Band mit drei starren Drähten, welches so angeordnet ist, dass es um die Brust eines Trägers des Kleidungsstücks verläuft. Die Drähte stellen Ausgangssignale bereit, die einer Auswerte-Elektronik zugeführt werden.

Die FR-A-2821262 offenbart eine Vorrichtung zum Bestimmen des Belastungszustands eines Individuums. Variationen des Atemvolumens werden als Funktion der Zeit gemessen und in ein elektrisches Signal umgewandelt, das die Atmungszyklen darstellt.

Die Aufgabe der vorliegenden Erfindung besteht somit darin, die genannten Nachteile des Stands der Technik zu beseitigen, d.h. den Tragekomfort von Messwertaufnehmern zu erhöhen und gleichzeitig eine Genauigkeit der Messung der Atmungsanstrengung zu erhöhen.

Diese Aufgabe wird durch ein Kleidungsstück mit den Merkmalen des Patentanspruchs 1 oder ein Verfahren zum Herstellen eines Kleidungsstücks nach Patentanspruch 10 gelöst.

Die Erkenntnis der vorliegenden Erfindung besteht darin, dass ein Verrutschen von in Gurten oder Bändern integrierten Sensoren zur Messung von Atmungsanstrengung verhindert werden kann, indem derartige Sensoren, wie beispielsweise induktiv arbeitende Sensoren, in ein Kleidungsstück integriert werden, welches über den Rumpf und insbesondere über den Thorax eines Lebewesens gezogen werden kann. Dabei ist der Sensor bei Ausführungsbeispielen der vorliegenden Erfindung eine elektrische Leiterstruktur, die in das Kleidungsstück derart integriert ist, so dass sich der Sensor bei angezogenem Kleidungsstück auf Höhe des Thorax befindet. Liegt das angezogene Kleidungsstück, wie z.B. ein T-Shirt oder ein Strampelanzug, relativ eng am Körper einer Person an, so kann ein Verrutschen des Sensors in Form des elektrischen Leiters weitgehend vermieden werden.

Gemäß einem Ausführungsbeispiel der vorliegenden Erfindung weist das Kleidungsstück eine Halterung für eine in das Kleidungsstück integrierbare Auswerte-Elektronik, die mit dem Sensor koppelbar ist, auf. Bei dieser Halterung kann es sich beispielsweise um eine Art Tasche handeln, in die die Auswerte-Elektronik, beispielsweise in Form einer integrierten Schaltung, eingesetzt werden kann. Die Auswerte-Elektronik ist ausgebildet, um von der elektrisch messbaren Eigenschaft abgeleitete Daten auszugeben oder zu speichern.

Durch die Atmungsanstrengung eines Lebewesens ändert sich der Durchmesser an verschiedenen Stellen seines Rumpfes und damit auch der Durchmesser des um den Rumpf geführten Sensors in Form eines elektrischen, in das Kleidungsstück integrierten Leiters. Durch diese Änderung des Durchmessers bzw. des Umfangs des elektrischen Leiters wird eine elektrisch messbare Eigenschaft des elektrischen Leiters in Abhängigkeit der Atmungsanstrengung des Thorax geändert. Ist die Auswerte-Elektronik mit dem elektrischen Leiter gekoppelt, so kann sie die elektrisch messbare Eigenschaft erfassen und über eine in die Auswerte-Elektronik integrierte Schnittstelle daraus abgeleitete Daten ausgeben oder speichern. Bei der Schnittstelle kann es sich um eine drahtgebundene oder vorzugsweise um eine drahtlose Schnittstelle zur Funkübertragung handeln.

Gemäß Ausführungsbeispielen ändert sich durch die Atmungsanstrengung die Induktivität des elektrischen Leiters, der bei angezogenem Kleidungsstück im Wesentlichen eine Leiterschleife um den Rumpf des Lebewesens bildet, wobei die Induktivität des elektrischen Leiters abhängig von dessen Durchmesser bzw. Umfang ist.

Bei anderen Beispielen ändert sich durch die Atmungsanstrengung der elektrische Widerstand des elektrischen Leiters. Dabei umfasst der elektrische Leiter vorzugsweise wenigstens eine elastische Faser, die bei Ausdehnung und Kontraktion jeweils ihren elektrischen Widerstand oder ihre Induktivität ändert.

Ausführungsbeispiele der vorliegenden Erfindung betreffen also ein eigenständiges Kleidungsstück, mit dem die Atmungsanstrengung und/oder daraus ableitbare Vitalparameter erfasst und bestimmt werden können. Weiterhin können diese Vitalparameter mittels der Auswerteelektronik in Echtzeit drahtlos übertragen oder wahlweise lokal gespeichert werden. Die Atmungsanstrengung kann an mindestens einer aber auch an mehreren Körperpartien gemessen werden. Im Falle von mehreren Körperpartien weist das Kleidungsstück gemäß einem Ausführungsbeispiel in Höhe des Abdomen (bei angezogenem Kleidungsstück) des Lebewesens einen weiteren elektrischen Leiter auf, der mit der Auswerte-Elektronik koppelbar ist.

Das Kleidungsstück ist für eine Überwachung von Neugeborenen, Kindern und Erwachsenen gleichermaßen geeignet. Sie ist zudem bequem und belastungsfrei. Zum Waschen kann die Auswerte-Elektronik und/oder deren Energieversorgung aus dem Kleidungsstück herausgenommen werden. Durch die in das Kleidungsstück integrierten Sensoren ist eine exakte Positionierung der Sensoren möglich. Zudem verrutschen die Sensoren nicht mehr während der Messung. Das hat den großen Vorteil, dass die Atmungsamplitude mit einer Einheit angegeben werden kann, beispielsweise Änderung eines Körperteilumfangs in cm oder Änderung des Körperteilvolumens in $cm^3$.

Durch die exakte Positionierung der elektrischen Leiter als Sensoren und durch die Fixierung dieser Sensoren an einer festgelegten Position ist eine genauere und zuverlässigere Messung der Atmungsbewegung möglich. Die Messung wird durch die feste Position zuverlässiger durch Verhindern des Verrutschens der elektrischen Leiter in eine Körperregion, in der physiologisch keine Atmung feststellbar ist. Durch das Wegfallen der Einflüsse, die durch ein Verrutschen der elektrischen Leiter entstehen, kann die gemessene Größe zudem mit einer Einheit angegeben werden.

Als zusätzlicher Vorteil ist das Anbringen der Messanordnung am Patienten zu sehen. Hier braucht das Kleidungsstück braucht einfach nur angezogen zu werden. Im Gegensatz dazu müssen herkömmliche Systeme, bestehend aus ihren Einzelteilen, positioniert, verdrahtet und an der Kleidung des Patienten befestigt werden.

Durch die exakte Positionierung der Sensoren sowie der daraus resultierenden genauen Messung der Umfangsänderung lassen sich beispielsweise Größen wie Atemfrequenz, Atemtiefe und vor allem Atemvolumen (mit vorheriger Kalibrierung) sehr genau bestimmen.

Bei Verwendung von elastischen, elektrischen Leitern bzw. Fasern, die gerade, d.h. kreis- bzw. ringförmig um den Thorax in das Kleidungsstück eingewebt sind, können zudem Tragekomfort und/oder Ästhetik verbessert werden. Eine derartige Faser kann sehr unauffällig in ein Kleidungsstück integriert werden und nimmt wenig Platz ein, wodurch eine Bewegungsfreiheit eines Trägers des Kleidungsstücks kaum beeinträchtigt wird. Durch unauffälliges Einarbeiten einer oder mehrerer elastischer Fasern kann das Kleidungsstück nicht von einem "normalen" eng anliegenden Kleidungsstück unterschieden werden, wodurch ein Außenstehender die Funktionalität des Kleidungsstücks, z. B. Überwachung von Vitalparametern, nicht erkennen kann. Somit sinkt unter Umständen auch eine Hemmschwelle, ein derartiges Kleidungsstück öffentlich zu tragen.

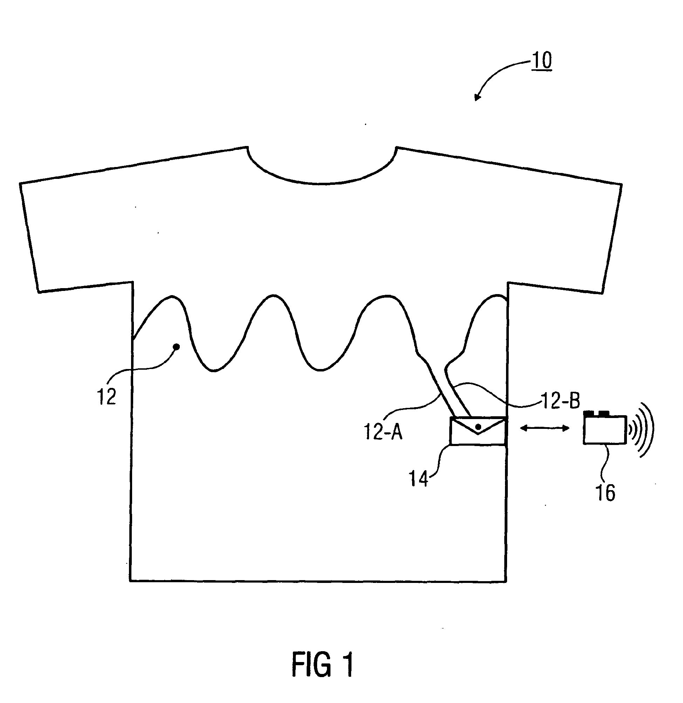

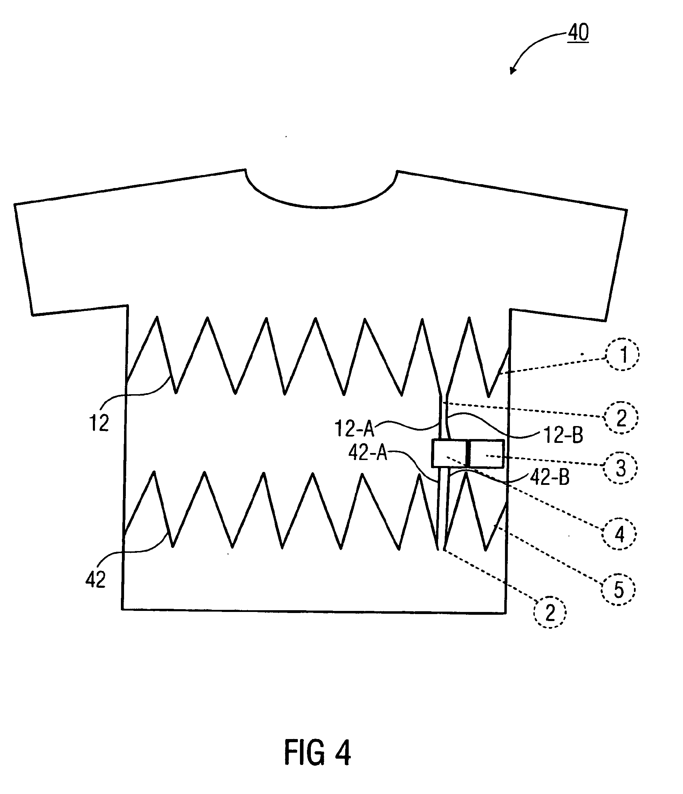

Ausführungsbeispiele der vorliegenden Erfindung werden nachfolgend bezugnehmend auf die beiliegenden Zeichnungen näher erläutert. Die Beispiele der Figuren 1, 4 7 und 8 sind kein Teil der Erfindung.

Fig. 1    eine schematische Darstellung eines Kleidungsstücks zur Erfassung einer Atembewegung

Fig. 2    eine Darstellung einer von einem magnetischen Fluss durchflossenen Leiterschleife;

Fig. 3    eine Darstellung einer Atemkurve aufgetragen über der Zeit;

Fig. 4    eine schematische Darstellung eines Kleidungsstücks zur Erfassung einer Atembewegung

Fig. 5    eine schematische Darstellung eines Kleidungsstücks zur Erfassung einer Atembewegung mit elastischen elektrischen Leitern gemäß einem Ausführungsbeispiel der vorliegenden Erfindung;

Fig. 6    eine Abbildung einer Auswerte-Elektronik gemäß einem Ausführungsbeispiel der vorliegenden Erfindung;

Fig. 7    eine Abbildung einer Person, welche ein Kleidungsstück trägt; und

Fig. 8    eine Abbildung eines Strampelanzugs für Babys .

Bezüglich der nachfolgenden Beschreibung sollte beachtet werden, dass bei den unterschiedlichen Ausführungsbeispielen gleich oder gleichwirkende Funktionselemente gleiche Bezugszeichen aufweisen und somit Beschreibungen dieser Funktionselemente in den verschiedenen in den nachfolgend dargestellten Ausführungsbeispielen untereinander austauschbar sind.

Fig. 1 zeigt eine schematische Darstellung eines Kleidungsstücks 10 zur Erfassung einer Atembewegung eines Lebewesens.

Obwohl im Nachfolgenden stets die Rede von der Erfassung der Atembewegung von menschlichen Lebewesen ist, sind Ausführungsbeispiele der vorliegenden Erfindung keineswegs nur auf Menschen beschränkt, sondern können in entsprechender Weise auf Tiere, insbesondere Säugetiere, angewendet werden.

Das Kleidungsstück 10 ist ausgebildet, um über den Thorax einer Person bzw. eines Lebewesens gezogen werden zu können. Bei bevorzugten Ausführungsbeispielen der vorliegenden Erfindung kann das Kleidungsstück 10 nicht nur über den Thorax, sondern über den kompletten Rumpf einer Person gezogen werden. Das Kleidungsstück kann beispielsweise als eng anliegendes T-Shirt oder, bei Kleinkindern, als eng anliegender Strampelanzug ausgebildet sein. Wichtig ist, dass das Kleidungsstück möglichst eng am Rumpf der Person anliegt. Dies kann beispielsweise dadurch erreicht werden, indem das Kleidungsstück 10 aus einem möglichst elastischen Gewebe besteht, welches sich bei der Atembewegung ausdehnen und wieder zusammenziehen kann. Derartige elastische Gewebe sind in einer Vielzahl verfügbar.

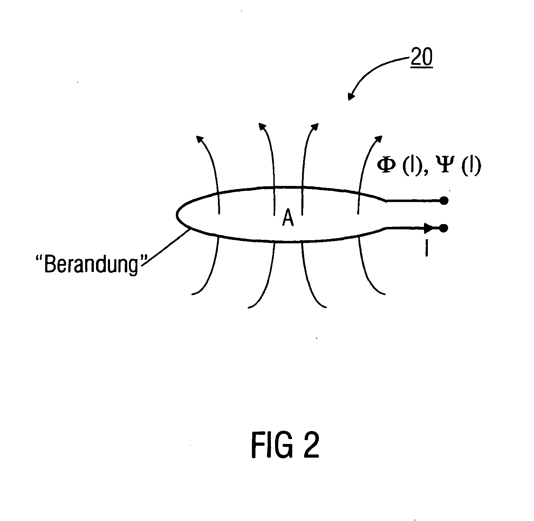

Stellt man sich das Kleidungsstück 10 über den Rumpf der Person gezogen vor, so umfasst das Kleidungsstück 10 in Höhe des Thorax der Person einen in das Kleidungsstück 10 integrierten Sensor in Form eines elektrischen Leiter 12, der so an dem Kleidungsstück 10 angebracht ist, dass sich in Abhängigkeit der Atembewegung des Thorax eine elektrisch messbare Eigenschaft des elektrischen Leiters 12 ändern kann. Der elektrische Leiter 12 ist dabei derart in das Kleidungsstück 10 integriert, dass der elektrische Leiter 12 von einem ersten Leitungsende 12-A bei angezogenem Kleidungsstück um den Thorax der Person herum reicht bis zu einem zweiten Leitungsende 12-B, ähnlich einer Leiterschleife, wie sie in Fig. 2 schematisch gezeigt ist.

Die beiden Leitungsenden 12-A, 12-B sind zu einer in das Kleidungsstück integrierten Halterung 14 geführt. Die Halterung 14 dient zum Befestigen einer in das Kleidungsstück integrierbaren Auswerte-Elektronik 16, die mit dem elektrischen Leiter 12 über die beiden Leitungsenden 12-A, 12-B koppelbar ist, und die ausgebildet ist, um die elektrisch messbare Eigenschaft des elektrischen Leiters 12 zu erfassen. Ferner weist die Auswerte-Elektronik eine Schnittstelle auf, um von der elektrisch messbaren Eigenschaft abgeleitete Daten auszugeben oder zu speichern. Bei der Schnittstelle der Auswerte-Elektronik 16 kann es sich, wie in Fig. 1 schematisch dargestellt, um eine drahtlose Schnittstelle zur Funkübertragung handeln. Ebenso ist natürlich eine drahtgebundene Schnittstelle oder eine Speicherschnittstelle denkbar.

Bei der Halterung 14 kann es sich gemäß Ausführungsbeispielen um eine Art Tasche handeln, in die die Auswerte-Elektronik eingesetzt und mit den beiden Leitungsenden 12-A, 12-B gekoppelt werden kann. Die Kopplung der Leitungsenden 12-A, 12-B mit der Auswerte-Elektronik 16 kann mittels einer Steckverbindung oder beispielsweise mittels Druckknöpfen erfolgen. Um die Auswerte-Elektronik 16 in der Tasche 14 zu fixieren, ist die Tasche 14 gemäß einem Ausführungsbeispiel der vorliegenden Erfindung mit einem Verschlussmechanismus versehen, wie beispielsweise einem Klett-Verschluss oder einem Druckknopf. Zum Waschen kann die Auswerte-Elektronik und/oder deren Energieversorgung wieder aus der Tasche genommen werden.

Alternativ könnte die Auswerte-Elektronik 16 auch wasserdicht ausgeführt sein, beispielsweise durch Eingießen der Auswerte-Elektronik 16 in ein Epoxidharz, und fest in das Kleidungsstück 10 integriert bzw. eingenäht sein. In diesem Fall ist die Halterung 14 für eine Energiequelle, wie z.B. einer Batterie oder einem Akku, für die Auswerte-Elektronik 16 am Kleidungsstück 10 vorzusehen.

Gemäß einem Beispiel handelt es sich bei dem elektrischen Leiter 12 um einen Draht, der isoliert sein kann, und der in das Kleidungsstück 10 eingenäht ist. Dabei kann der isolierte Draht 12 auf verschiedene Weisen in das Kleidungsstück 10 eingenäht sein. Der Draht 12 kann beispielsweise direkt durch das Stoffgewebe des Kleidungsstücks 10 laufen, oder aber auf den Stoff in einer extra dafür vorgesehenen Lasche verlaufen. Um der Atembewegung des Thorax der Person folgen zu können, d.h. sich ausdehnen und zusammenziehen zu können, ist der elektrische Leiter 12 beispielsweise sinusförmig, mäander-förmig oder zick-zack-förmig in das Kleidungsstück 10 integriert.

Dadurch, dass der elektrische Leiter 12 bei angezogenem Kleidungsstück um den Thorax der Person herumläuft, bildet er eine Leiterschleife, welche schematisch in Fig. 2 gezeigt ist.

Bei Anlegen eines Signals, z.B. in Form eines Stroms oder einer Spannung, an die beiden Leiterenden 12-A, 12-B der Leiterschleife 12 entsteht ein magnetisches Feld und damit ein magnetischer Fluss. Das Verhältnis, gebildet aus dem magnetischen Fluss durch die Leiterschleife, zum Strom in der Leiterschleife, nennt man Induktivität L. Unter der Voraussetzung, dass der Durchmesser d des verwendeten elektrischen Leiters 12 sehr klein gegenüber dem Durchmesser D der durch den elektrischen Leiter 12 gebildeten Leiterschleife ist (d/D < 0,001), d.h. dem Durchmesser des Thorax, kann eine relativ einfache Nährungslösung für die Induktivität der Leiterschleife verwendet werden. Demnach ergibt sich die Induktivität L zu

$$L = \mu_0 \cdot R \cdot \ln(2R/d).$$

Dabei ist R = D/2 der Radius der Leiterschleife, d der Durchmesser des verwendeten elektrischen Leiters 12 und $\mu_0$ ist die magnetische Feldkonstante.

Durch die Atembewegung des Thorax verändert sich der Radius bzw. der Umfang der eng an dem Thorax anliegenden Leiterschleife 12 und damit deren Induktivität L. Die mit der Atembewegung veränderliche Induktivität L des elektrischen Leiters 12 bildet gemäß einem Ausführungsbeispiel der vorliegenden Erfindung die Induktivität eines LC-Parallelschwingkreises. Gemäß einem Ausführungsbeispiel der vorliegenden Erfindung handelt es sich bei dem LC-Parallelschwingkreis um einen Teil einer unter dem Namen Colpitts-Schaltung bzw. Colpitts-Oszillator bekannten elektrischen Schaltung. Da lediglich die Induktivität L des elektrischen Leiters 12 variiert, wird durch sie im Wesentlichen die durch den Colpitts-Oszillator erzeugte Frequenz bestimmt. Der Colpitts-Oszillator befindet sich dabei, bis auf den elektrischen Leiter 12, in der Auswerte-Elektronik 16. Somit kann die Atembewegung der Person über die veränderliche Induktivität L des elektrischen Leiters 12 in eine von der die Atembewegung der Person abhängige Frequenz der Oszillatorschaltung umgesetzt werden.

Gemäß Ausführungsbeispielen der vorliegenden Erfindung umfasst die Auswerte-Elektronik 16 einen Frequenzzähler, um die durch die Atmungsanstrengung variierende Frequenz der Oszillatorschaltung zu erfassen. Dehnt sich der Thorax des Lebewesens beim Einatmen aus, so erhöht sich die Induktivität L des elektrischen Leiters 12 aufgrund des zunehmenden Radius R der durch den elektrischen Leiter 12 gebildeten Leiterschleife um den Thorax. Damit verringert sich die von der Oszillatorschaltung mit dem LC-Parallelschwingkreis erzeugte Frequenz. Umgekehrtes gilt beim Ausatmen.

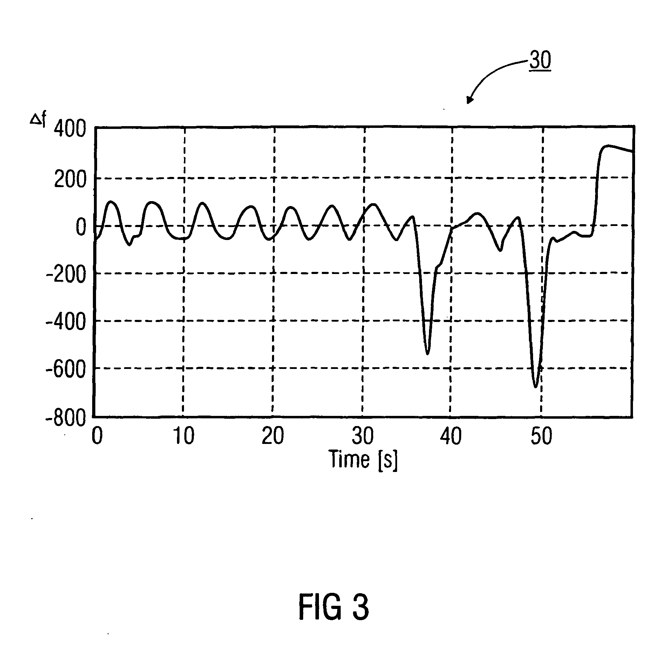

Durch Überwachung der Frequenz des Colpitts-Oszillators kann eine von der Atembewegung der Person abhängige Atemkurve dargestellt werden. Eine derartige Atemkurve ist beispielhaft in Fig. 3 gezeigt.

Bei der in Fig. 3 dargestellten Atemkurve 30 ist eine Thoraxumfangsabweichung $\Delta U$ von einem Nominalthoraxumfang über der Zeit aufgetragen. Es lässt sich erkennen, dass bis zu einer Zeitdauer von etwa 35 Sekunden eine relativ normale, gleichmäßige Atembewegung vorherrscht. Zum Zeitpunkt bei etwa 35 Sekunden ist jedoch ein Einbruch der Atemkurve erkennbar, was einem starken Ausatmen zuzurechnen ist. In einem Zeitraum zwischen 40 und ca. 47 Sekunden normalisiert sich die Atemkurve 30 wieder, um dann bei ca. 50 Sekunden einen erneuten Einbruch zu erleiden.

Es wird deutlich, dass mit dem hier vorgestellten Konzept eine relativ genaue und zuverlässige Überwachung der Atmungsaktivität eines Lebewesens erreicht werden kann.

Fig. 4 zeigt eine schematische Darstellung eines Beispiels eines Kleidungsstücks zur Erfassung einer Atembewegung einer Person.

Um die Erfassung der Atmungsaktivität noch zuverlässiger zu gestalten, weist das Kleidungsstück 40 gegenüber dem in Fig. 1 gezeigten Kleidungsstück 10 zusätzlich zu dem elektrischen Leiter 12 einen weiteren elektrischen Leiter 42 auf, der so in das Kleidungsstück 40 integriert ist, um in Abhängigkeit der Atembewegung des Abdomen eine elektrisch messbare Eigenschaft zu ändern. Gemäß Ausführungsbeispielen handelt es sich bei der elektrisch messbaren Eigenschaft des weiteren Leiters 42 ebenfalls um dessen Induktivität. Der weitere Leiter 42 ist ebenfalls über seine Leitungsenden 42-A, 42-B mit der Auswerte-Elektronik 16 koppelbar. Der weitere Leiter 42 ist gemäß Ausführungsbeispielen auf die gleiche Art und Weise in das Kleidungsstück 40 integriert wie der erste elektrische Leiter 12.

Gemäß Ausführungsbeispielen kann der weitere elektrische Leiter 42 über seine Leitungsenden 42-A, 42-B mit einer weiteren Colpitts-Oszillatorschaltung gekoppelt werden, um einen weiteren Frequenzverlauf abhängig von der Atembewegung des Lebewesens zu erhalten. Der Frequenzverlauf kann weiters in andere Vitalparameter, wie z.B. Körperteilumfang usw., umgerechnet werden. Durch den weiteren elektrischen Leiter 42 kann die Zuverlässigkeit des Messverfahrens weiter gesteigert werden.

Die beiden elektrischen Leiter 12, 42 sind um den gesamten Rumpf in einer vordefinierten Höhe beispielsweise als Einzelleiter eingewebt oder als Litze eingenäht. Handelt es sich um gewöhnliche, nicht elastische elektrische Leitungen, wie z.B. metallische Drähte, ist eine zick-zack-förmige, sinusförmige oder mäander-förmige Anordnung der Leitungen vorteilhaft, um der Atembewegung Rechnung tragen zu können. Die elektrischen Leitungen 12, 42 können jeweils an einer beliebigen Stelle unterbrochen werden. Diese Unterbrechung resultiert in den jeweiligen Leitungsenden und dient als Anschluss für die Auswerte-Elektronik 16.

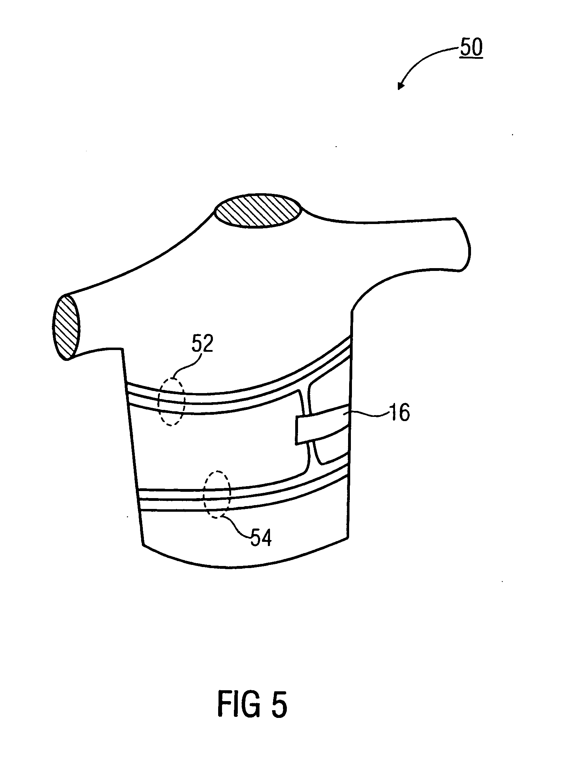

Gemäß der vorliegenden Erfindung werden als elektrische Leiter 12 und/oder 42 elastische Fasern verwendet , die bei Ausdehnung beispielsweise ihren elektrischen Widerstand R oder ihre Induktivität L verändern. Bei der Verwendung solcher elastischer Fasern brauchen diese nicht zick-zack-förmig, sinusförmig oder mäander-förmig in das Kleidungsstück integriert werden, sondern können gerade, d.h. kreis- bzw. ringförming um den Thorax, eingewebt werden, so wie es schematisch in Fig. 5 gezeigt ist. Dabei kann über die Anzahl benachbart verlaufender elastischer elektrischer Leiter 52, 54 bzw. der Anzahl der Windungen der elastischen elektrischen Leiter 52, 54 ein Nominalwiderstand eingestellt werden, der an die Auswerte-Elektronik 16 angepasst ist. Durch die Atembewegung des Thorax bzw. des Abdomen verändern sich z.B. jeweils die elektrischen Widerstände der dehnbaren elektrischen Leiter 52, 54, so dass in diesem Fall beispielsweise ein im vorhergehenden beschriebener LC-Parallelschwingkreis variabel bedämpft werden kann. Eine Widerstandsänderung kann auf zahlreiche, dem Fachmann bekannte Weisen detektiert und auf ein Signal, ähnlich dem in Fig. 3 gezeigten, abgebildet werden.

Durch Verwendung eines elastischen elektrischen Leiters, der gerade, d.h. kreis- bzw. ringförmig um den Thorax in das Kleidungsstück eingewebt ist, kann der Tragekomfort und die Ästhetik wesentlich verbessert werden. Erfindungsgemäß wird als elektrischer Leiter eine elektrisch leitfähige, elastische Faser verwendet. Eine derartige Faser kann wesentlich unauffälliger in das Kleidungsstück integriert werden, als ein kurvenförmig verlaufender Draht. Eine derartige Faser nimmt weniger Platz ein, wodurch die Bewegungsfreiheit des Trägers des Kleidungsstücks weniger beeinträchtigt wird. Das heißt, der Tragekomfort wird erhöht. Durch das unauffällige Einarbeiten einer oder mehrerer elastischer Fasern kann das Kleidungsstück nicht von einem "normalen" eng anliegenden Kleidungsstück unterschieden werden, wodurch ein Außenstehender die Funktionalität des Kleidungsstücks, z. B. zur Messung von Vitalparametern, nicht erkennen kann. Somit sinkt unter Umständen auch die Hemmschwelle eines Patienten, ein derartiges Kleidungsstück in der Öffentlichkeit zu tragen.

Die Auswerte-Elektronik 16 dient zum Erfassen und Auswerten der Messwerte der Sensoren 12, 42, dem Aufbereiten der erfassten Signale und dem Bereitstellen der Daten über eine drahtlose oder drahtgebundene Schnittstelle. Alternativ können die erfassten Daten auch gespeichert werden. Eine Energiequelle, beispielsweise ein Akku, versorgt die Auswerte-Elektronik 16 mit Energie. Die Kleidung 10, 40 sollte auf das Lebewesen bzw. den Probanden angepasst sein und sollte dabei eng anliegen, wie z.B. ein Unterhemd oder ein Sport-Shirt.

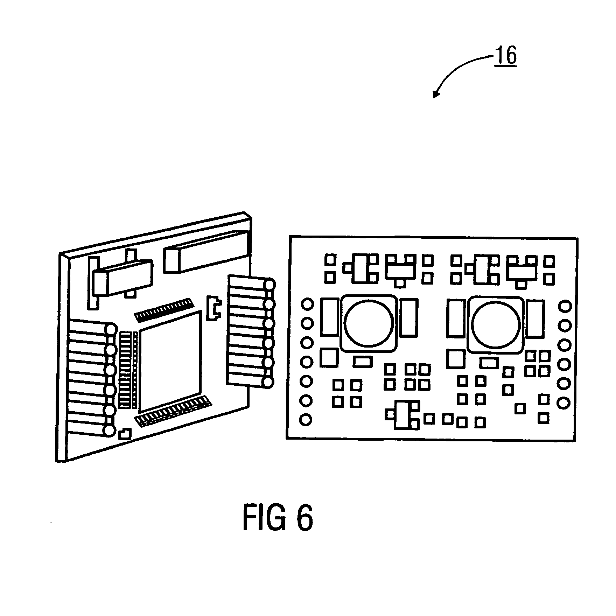

Eine Auswerte-Elektronik 16 gemäß einem Ausführungsbeispiel der vorliegenden Erfindung ist in Fig. 6 abgebildet.

Die Auswerte-Elektronik 16 ist auf einer kleinen und leichten Platine untergebracht. Gemäß einer Ausführungsform weist die Platine eine Abmessung von ca. 2 x 2 cm auf und ist somit leicht in das Kleidungsstück integrierbar. Wie im vorhergehenden bereits beschrieben wurde, kann die Auswerte-Elektronik beispielsweise eine Colpitts-Schaltung aufweisen, wobei die Induktivität des LC-Parallelschwingkreises von dem elektrischen Leiter gebildet wird. In diesem Fall weist die Auswerte-Elektronik 16 einen Frequenzzähler auf, um die von dem Colpitts-Oszillator erzeugte Frequenz zu erfassen.

Statt eine sich in Abhängigkeit der Atembewegung verändernde Induktivität des elektrischen Leiters zu erfassen, kann die Auswerte-Elektronik 16 gemäß Beispielen auch ausgebildet sein, um beispielsweise eine Änderung des elektrischen Widerstands R eines elektrischen Leiters zu erfassen. Dies kann beispielsweise dann vorgesehen sein, wenn elastische bzw. dehnbare Fasern als elektrische Leiter verwendet werden, die ihren elektrischen Widerstand R in Abhängigkeit von ihrer Ausdehnung ändern.

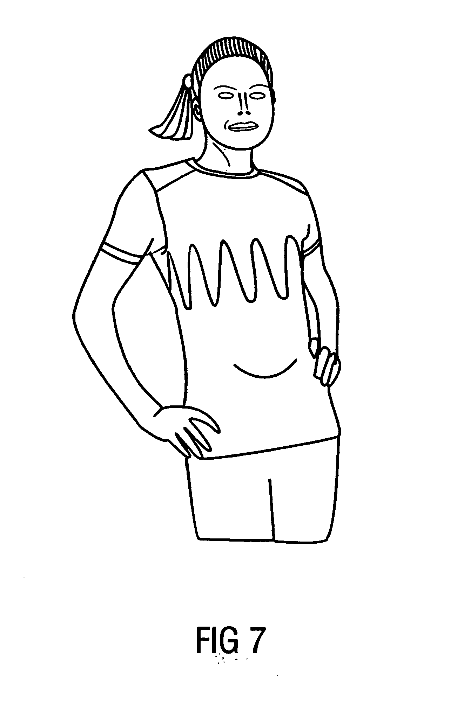

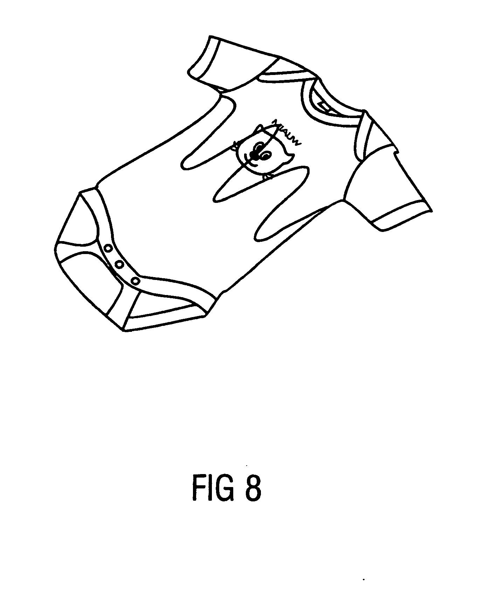

Beispielhaft sind in den Figuren 7 und 8 jeweils zwei Kleidungsstücke abgebildet.

Fig. 7 zeigt ein Sport-Shirt mit integrierten elektrischen Leitern als Messwertaufnehmer und integrierter Auswerte-Elektronik. Dabei ist zu bemerken, dass das Sport-Shirt relativ eng am Rumpf der Trägerin anliegt.

Fig. 8 zeigt einen Baby-Strampelanzug mit integrierten elektrischen Leitern als Messwertaufnehmer. Wie eingangs bereit erwähnt wurde, kann durch die Verwendung eines solchen Baby-Strampelanzugs mit integrierter Atmungsdetektion ein plötzlicher Kindstod erkannt und verhindert werden.

Das erfindungsgemäße Konzept beruht maßgeblich auf einer Detektion einer Änderung des Umfangs eines eingewebten bzw. eingenähten elektrischen Leiters. Diese Änderung des Umfangs bewirkt eine Änderung der Induktivität L oder des Widerstands R des elektrischen Leiters. Die Induktivität L bzw. der Widerstand R können mittels der Auswerte-Elektronik kontinuierlich erfasst und digitalisiert werden.

Mit einem Gesamtsystem, welches ein Kleidungsstück mit integriertem elektrischem Leiter und daran angeschlossener Auswerte-Elektronik umfasst, ist eine kontinuierliche Messung des Körperumfangs an mehreren festgelegten Stellen, jedoch mindestens einer, möglich. Durch die Fixierung der elektrischen Leiter in dem Kleidungsstück ändern sich die Messorte bei Benutzung nicht. Damit ist eine exakte Messung der Atmungsanstrengung und der daraus ableitbaren Vitalparameter möglich.

Zur Auswertung der aufgenommen Signale dient gemäß Ausführungsbeispielen der vorliegenden Erfindung eine Auswerte-Elektronik in Form einer kleinen Platine, die ebenfalls in das Kleidungsstück integrierbar ist. Gemäß Ausführungsbeispielen weist die Platine eine Funkschnittstelle auf, um die Messwerte per Funk an eine Anzeige-, Alarmierungs- und/oder Aufzeichnungs-Einheit zu übermitteln. Dies kann beispielsweise eine Armbanduhr, ein PDA (Personal Digital Assistant) oder ein PC (Personal Computer) sein. Bei medizinischen Anwendungen kann bei Atemaussetzern Alarm ausgelöst werden. Im Sportumfeld kann beispielsweise durch das erfindungsgemäße Konzept die Atemfrequenz ermittelt werden und sämtliche Daten für eine spätere Analyse aufgezeichnet werden.

Zusammenfassend soll darauf hingewiesen werden, dass die vorliegende Erfindung nicht auf die jeweiligen Einzelteile des Kleidungsstücks oder die erläuterte Vorgehensweise beschränkt ist, da diese Einzelteile und Verfahren variieren können. Die verwendeten Begriffe sind lediglich dafür bestimmt, besondere Ausführungsformen zu beschreiben und werden nicht einschränkend verwendet. Wenn in der Beschreibung und in den Ansprüchen die Einzahl oder unbestimmte Artikel verwendet werden, beziehen sich diese auch auf die Mehrzahl dieser Elemente, solange nicht der Gesamtzusammenhang nicht etwas anderes deutlich macht. Dasselbe gilt in umgekehrter Richtung.

**Patentansprüche**

1. Kleidungsstück (10; 40) zur Erfassung einer Atembewegung eines Lebewesens, wobei das Kleidungsstück über den Thorax des Lebewesens gezogen werden kann, mit folgenden Merkmalen:

   einem in Höhe des Thorax des Lebewesens in das Kleidungsstück (10; 40) integrierbaren geraden elektrischen Leiter (12), der so an dem Kleidungsstück angebracht ist, um in Abhängigkeit der Atembewegung des Thorax eine Induktivität (L) des elektrischen Leiters (12) zu ändern, wobei der elektrische Leiter eine elastische elektrisch leitfähige Faser ist, die sich abhängig von der Atembewegung ausdehnen kann und dabei eine Änderung der Induktivität (L) erfährt, wenn das Kleidungsstück über den Thorax des Lebewesens gezogen ist;
   einer Halterung (14) zum Befestigen einer in das Kleidungsstück (10; 40) integrierbaren Auswerte-Elektronik (16); und
   die integrierbare Auswerte-Elektronik (16), die mit der elastischen elektrisch leitfähigen Faser (12) koppelbar ist und die ausgebildet ist, um eine Änderung der Induktivität (L) der elastischen elektrisch leitfähigen Faser (12) zu erfassen, und die ferner eine Schnittstelle aufweist, um von der Induktivität (L) abgeleitete Daten auszugeben oder zu speichern.

2. Kleidungsstück gemäß Anspruch 1, wobei die integrierbare Auswerte-Elektronik (16) so ausgebildet ist, dass die mit der Atembewegung veränderliche Induktivität (L) der elastischen elektrisch leitfähigen Faser eine Induktivität eines LC-Parallelschwingkreises bildet, der Teil einer Colpitts-Oszillatorschaltung ist, wobei sich die Colpitts-Oszillatorschaltung in der integrierbaren Auswerte-Elektronik (16) befindet.

3. Kleidungsstück gemäß Anspruch 2, wobei die integrierbare Auswerte-Elektronik (16) einen Frequenzzähler umfasst, um eine durch die Atembewegung variierende Frequenz der Colpitts-Oszillatorschaltung zu erfassen.

4. Kleidungsstück gemäß einem der Ansprüche 1, bis 3 wobei der elektrische Leiter (12), so in das Kleidungsstück integriert ist, dass eine von der elastischen elektrisch leitfähigen Faser umschlossene Fläche abhängig von der Atembewegung ist, wenn das Kleidungsstück über den Thorax des Lebewesens gezogen ist.

5. Kleidungsstück gemäß einem der vorhergehenden Ansprüche, wobei in der Halterung (14) die Auswerte-Elektronik (16) derart befestigt ist dass die Auswerte-Elektronik (16) Bestandteil des Kleidungsstücks ist, wobei die Auswerte-Elektronik (16) wasserfest ausgebildet ist.

6. Kleidungsstück gemäß einem der vorhergehenden Ansprüche, wobei die integrierbare Auswerte-Elektronik (16) eine drahtlose Schnittstelle zur Funkkommunikation aufweist.

7. Kleidungsstück gemäß einem der vorhergehenden Ansprüche, wobei ein Gewebe des Kleidungsstücks elastisch ist, um ein enges Anliegen des elektrischen Leiters am Rumpf des Lebewesens zu ermöglichen.

8. Kleidungsstück gemäß einem der vorhergehenden Ansprüche, wobei das Kleidungsstück zusätzlich über den Abdomen des Lebewesens gezogen werden kann, und in Höhe des Abdomens des Lebewesens einen weiteren elektrischen Leiter (42) aufweist, der so an dem Kleidungsstück (10; 40) angebracht ist, um in Abhängigkeit der Atembewegung des Abdomens eine elektrisch messbare Eigenschaft (L; R) zu ändern, und wobei der weitere elektrische Leiter (42) mit der integrierbaren Auswerte-Elektronik (16) koppelbar ist.

9. Verwendung eines Kleidungsstücks gemäß einem der vorhergehenden Ansprüche zur Erfassung einer Atembewegung eines Lebewesens, wobei mittels der Halterung (14) die integrierbare Auswerte-Elektronik (16) an dem Kleidungsstück befestigt ist, wobei die integrierbare Auswerte-Elektronik (16) mit der elastischen elektrisch-leitfähigen Faser gekoppelt ist, um die Änderung der Induktivität (L) der elastischen elektrisch leitfähigen Faser (12) zu erfassen.

10. Verfahren zum Herstellen eines Kleidungsstücks (10; 40) zur Erfassung einer Atembewegung eines Lebewesens, wobei das Kleidungsstück über den Thorax des Lebewesens gezogen werden, mit folgenden folgenden Schritten:

Integrieren einer elastischen, elektrisch leitfähigen Faser (12) in das Kleidungsstück (10; 40), so dass sich die elastische, elektrisch leitfähige Faser (12) in Abhängigkeit der Atembewegung des Thorax ausdehnen kann, wenn das Kleidungsstück über den Thorax des Lebewesens gezogen ist, und dabei eine Änderung einer Induktivität (L) der elastischen, elektrisch leitfähigen Faser (12) erfährt; und

Anbringen, an dem Kleidungsstück, einer Halterung (14) zum Befestigen einer in das Kleidungsstück (10; 40) integrierbaren Auswerte-Elektronik (16); und

Bereitstellen der integrierbaren Auswerte-Elektronik (16) die mit der elastischen, elektrisch leitfähigen Faser (12) koppelbar ist und die ausgebildet ist, um

eine Änderung der Induktivität (L) der elastischen, elektrisch leitfähigen Faser (12) zu erfassen, und die ferner eine Schnittstelle aufweist, um von der Induktivität abgeleitete Daten auszugeben oder zu speichern.

**Claims**

1. Garment (10; 40) for detecting respiratory movement of a living being, wherein the garment can be pulled over the thorax of the living being, comprising:

a straight electric conductor (12), which is integrable into the garment (10; 40) at the height of the thorax of the living being, which is attached to the garment in order to change an inductance (L) of the electric conductor (12) in dependence on the respiratory movement of the thorax, wherein the electric conductor is an elastic electrically conductive fiber, which can expand in dependence on the respiratory movement and thereby experiences a change of the inductance (L) when the garment is pulled over the thorax of the living being; and
a holder (14) for fixing evaluation electronics (16), which is integrable into the garment (10; 40); and
the integrable evaluation electronics (16), which can be coupled to the elastic electrically conductive fiber (12) and is implemented to detect a change in inductance (L) of the elastic electrically conductive fiber (12) and further comprises an interface for outputting or storing the data derived from the inductance (L).

2. Garment according to claim 1, wherein the integrable evaluation electronics (16) is implemented such that the inductance (L) of the elastic electrically conductive fiber variable with the respiratory movement forms an inductance of an LC parallel resonant circuit, which is part of a Colpitts oscillator circuit, wherein the Colpitts oscillator circuit is within the integrable evaluation electronics (16).

3. Garment according to claim 2, wherein the integrable evaluation electronics (16) comprises a frequency counter for detecting a frequency of the Colpitts oscillator circuit varying due to the respiratory movement.

4. Garment according to one of claims 1 to 3, wherein the electric conductor (12) is integrated into the garment such that an area surrounded by the elastic electrically conductive fiber is dependent on the respiratory movement when the garment is pulled over the thorax of the living being.

5. Garment according to one of the previous claims, wherein the evaluation electronics (16) is fixed in the holder (14) such that the evaluation electronics (16) is part of the garment, wherein the evaluation electronics (16) is implemented in a watertight manner.

6. Garment according to one of the previous claims, wherein the integrable evaluation electronics (16) comprises a wireless interface for radio communication.

7. Garment according to one of the previous claims, wherein a fabric of the garment is elastic for allowing tight application of the electric conductor to the torso of the living being.

**8.** Garment according to one of the previous claims, wherein the garment can additionally be pulled over the abdomen of the living being and comprises a further electric conductor (42) at the height of the abdomen of the living being, which is attached to the garment (10; 40) in order to change an electrically measurable characteristic (L; R) in dependence on the respiratory movement of the abdomen and wherein the further electric conductor (42) can be coupled to the integrable evaluation electronics (16).

**9.** Usage of a garment according to one of the previous claims for detecting respiratory movement of a living being, wherein the integrable evaluation electronics (16) is fixed to the garment by means of the holder (14), wherein the integrable evaluation electronics (16) is coupled to the elastic electrically conductive fiber for detecting the change in inductance (L) of the elastic electrically conductive fiber (12).

**10.** Method for producing a garment (10; 40) for detecting respiratory movement of a living being, wherein the garment can be pulled over the thorax of the living being, comprising:

integrating an elastic electrically conductive fiber (12) into the garment (10; 40), such that the elastic electrically conductive fiber can expand in dependence on the respiratory movement of the thorax when the garment is pulled over the thorax of the living being and thereby experiences change of an inductance (L) of the elastic electrically conductive fiber (12); and

attaching a holder (14) to the garment for fixing evaluation electronics (16) integrable into the garment (10; 40); and

providing the integrable evaluation electronics (16), which can be coupled to the elastic electrically conductive fiber (12) and which is implemented to detect a change in inductance (L) of the elastic electrically conductive fiber (12) and which further comprises an interface for outputting or storing data derived from the inductance.

**Revendications**

**1.** Pièce d'habillement (10; 40) à détection d'un mouvement respiratoire d'un être vivant, la pièce d'habillement pouvant être enfilée sur le thorax de l'être vivant, aux caractéristiques suivantes:

un conducteur électrique droit (12) intégrable, à hauteur du thorax de l'être vivant, dans la pièce d'habillement (10; 40), lequel est placé dans la pièce d'habillement, pour modifier, en fonction du mouvement respiratoire du thorax, une inductance (L) du conducteur électrique (12), le conducteur électrique étant une fibre électro-conductrice élastique pouvant s'étirer en fonction du mouvement respiratoire et subissant un changement de l'inductance (L) lorsque la pièce d'habillement est enfilée sur le thorax de l'être vivant;

un support (14) destiné à fixer une électronique d'évaluation (16) intégrable dans la pièce d'habillement (10; 40); et

l'électronique d'évaluation intégrable (16) qui peut être couplée à la fibre électro-conductrice élastique (12) et qui est réalisée pour saisir un changement de l'inductance (L) de la fibre électro-conductrice élastique (12), et qui présente par ailleurs une interface, pour sortir ou mémoriser les données dérivées de l'inductance (L).

**2.** Pièce d'habillement selon la revendication 1, dans laquelle l'électronique d'évaluation intégrable (16) est réalisée de sorte que l'inductance (L), variable avec le mouvement respiratoire, de la fibre électro-conductrice élastique constitue par ailleurs une inductance d'in circuit oscillant parallèle LC qui fait partie d'un circuit oscillant de Colpitts, le circuit oscillant de Colpitts se trouvant dans l'électronique d'évaluation intégrable.

**3.** Pièce d'habillement selon la revendication 2, dans laquelle l'électronique d'évaluation intégrable (16) comporte un compteur de fréquence, pour saisir une fréquence, variable par le mouvement respiratoire, du circuit oscillant de Colpitts.

**4.** Pièce d'habillement selon l'une des revendications 1 à 3, dans laquelle le conducteur électrique (12) est intégré dans la pièce d'habillement de sorte qu'une face entourée par la fibre électro-conductrice élastique soit fonction du mouvement respiratoire lorsque la pièce d'habillement est enfilée sur le thorax de l'être vivant.

**5.** Pièce d'habillement selon l'une des revendications précédentes, dans laquelle l'électronique d'évaluation (16) est fixée dans le support (14) de sorte que l'électronique d'évaluation (16) fasse partie de la pièce d'habillement, l'électronique d'évaluation (16) étant réalisée étanche à l'eau.

**6.** Pièce d'habillement selon l'une des revendications précédentes, dans laquelle l'électronique d'évaluation intégrable (16) présente une interface sans fil pour la communication radioélectrique.

**7.** Pièce d'habillement selon l'une des revendications précédentes, dans laquelle un tissu de la pièce d'habillement est élastique, pour permettre une application étroite du conducteur électrique contre le tronc de l'être vivant.

8. Pièce d'habillement selon l'une des revendications précédentes, dans laquelle la pièce d'habillement peut en outre être enfilée sur l'abdomen de l'être vivant et présente, à hauteur de l'abdomen de l'être vivant, un autre conducteur électrique qui est placé sur la pièce d'habillement (10; 40) de manière à modifier, en fonction du mouvement respiratoire de l'abdomen, une propriété mesurable électriquement (L; R), et dans laquelle l'autre conducteur électrique (42) peut être couplé à l'électronique d'évaluation intégrable (16).

9. Utilisation d'une pièce d'habillement selon l'une des revendications précédentes pour saisir un mouvement respiratoire d'un être vivant, dans laquelle l'électronique d'évaluation intégrable (16) est fixée à la pièce d'habillement au moyen du support (14), dans laquelle l'électronique d'évaluation intégrable (16) est couplée à la fibre électro-conductrice élastique, pour saisir le changement de l'inductance (L) de la fibre électro-conductrice élastique (12).

10. Procédé de fabrication d'une pièce d'habillement (10; 40) pour saisir un mouvement respiratoire d'un être vivant, la pièce d'habillement pouvant être enfilée sur le thorax de l'être vivant, aux étapes suivantes consistant à :

   intégrer une fibre électro-conductrice élastique (12) dans la pièce d'habillement (10; 40), de sorte que la fibre électro-conductrice élastique (12) puisse s'étirer en fonction du mouvement respiratoire du thorax lorsque la pièce d'habillement est enfilée sur le thorax de l'être vivant et subisse une modification d'une inductance (L) de la fibre électro-conductrice élastique (12); et
   placer sur la pièce d'habillement un support (14) pour fixer une électronique d'évaluation intégrable (16) dans la pièce d'habillement (10; 40); et
   préparer l'électronique d'évaluation intégrable 16) qui peut être couplée à la fibre électro-conductrice élastique (12) et qui est réalisée pour saisir un changement de l'inductance (L) de la fibre électro-conductrice élastique (12), et qui présente par ailleurs une interface, pour sortir ou mémoriser les données dérivées de l'inductance.

FIG 1

$\underline{20}$

$\Phi$ (I), $\Psi$ (I)

A

"Berandung"

I

FIG 2

FIG 3

FIG 4

FIG 5

FIG 6

FIG 7

FIG 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1731094 A
- US 6341504 B1
- FR 2821262 A